# EUROPEAN PATENT APPLICATION

(11) **EP 4 372 024 A1**
(43) Date of publication of application: **22.05.2024**
(21) Application number: 22207416.3
(22) Date of filing: 15.11.2022
(51) Int. Cl.: C08G 18/09, C08G 18/16, C08G 18/18, C08G 18/22, C08G 18/42, C08G 18/76, C08J 9/14, C08J 11/14, C08J 11/16, C08J 11/28

(54) **NEW METHOD FOR RECYCLING OF POLYISOCYANURATES**

(71) Applicant: Evonik Operations GmbH, 45128 Essen (DE)
(72) Inventor: OTTO, Sarah, 45128 Essen (DE); SUCHAN, Michael, 45894 Gelsenkirchen (DE); FERENZ, Michael, 45147 Essen (DE); WEISS, Christine, 46282 Dorsten (DE)
(74) Representative: Evonik Patent Association

(57) **Abstract**

The present invention relates to a process for depolymerization of polyisocyanurate, especially flexible and rigid polyisocyanurate foams, wherein valuable raw materials of the polyisocyanurate can be recovered in high yields and good quality, i.e. a quality allowing usage of the recycled raw materials for production of new polyurethanes, preferably polyurethane foams, or polyisocyanurates, preferably polyisocyanurate foams.

## Description

The present invention relates to a process for depolymerization of polyisocyanurate, especially rigid polyisocyanurate foams, wherein valuable raw materials of the polyisocyanurate can be recovered in high yields and good quality, i.e. a quality allowing usage of the recycled raw materials for production of new chemicals, in particular new polyurethanes, preferably polyurethane foam, or polyisocyanurates, preferably polyisocyanurate foams.

Polyurethanes are materials of considerable utility in the production of rigid and flexible foams, solid and microcellular elastomers, sealants, coatings, and adhesives. The versatility, relatively low cost, and superior properties of polyurethanes have resulted in the rapid growth of the polyurethane industry over the past 50 years. Currently, many thousand tons of polyurethanes are produced each year throughout the world and different methods have been developed for chemical recycling of polyurethanes, e.g. glycolysis, ammonolysis, aminolysis, acidolysis and hydrolysis. These methods have in common that mainly urethane and urea groups but also adjacent uretdione, carbodiimide, allophanate, biuret groups are cleaved.

Polyisocyanurate polymers differ from polyurethanes in that they comprise isocyanurate groups which are more stable and less reactive than the functional groups cleaved during chemolysis of polyurethanes. Another difference between polyurethanes and polyisocyanurates is that polyurethanes are usually formed by reaction of a polyisocyanates and a polyetherpolyol while polyisocyanurates are obtained from a reaction of a polyisocyanates and a polyesterpolyol. In contrast to polyether groups, polyester groups are cleaved during chemolysis, too. Thus, the chemical methods developed and used for polyurethane recycling cannot be applied to polyisocyanurates.

Because of the higher chemical stability, polyisocyanurate foams are used as insulation plates in applications with particularly high requirements on strength and flame retardancy. It has long been known to those skilled in the art that high chemical stability of polyisocyanurates can be achieved by a high number of isocyanurate groups. The prevailing opinion in the art was that polyisocyanurate foams cannot be cleaved in recycling processes based on chemical recycling, especially hydrolysis, to recover the raw materials of the polyisocyanurate foams, and that the isocyanurate structure is largely preserved.

P.N. Gribkova et al, "Degradation of a polyisocyanurate obtained by polycyclotrimerization of 4,4'-di-isocyanatodiphenylmethan", Polymer Science U.S.S.R., Vol. 22, pp. 299 - 304, compares thermal, thermal-hydrolytic and thermal-oxidative degradation of isocyanurates. It was found that under all conditions homolytic decomposition and formation of CO and H₂ was observed, and no high yield of raw materials was obtained. This confirms the prevailing opinion in the art that polyisocyanurates cannot be recycled to monomers that can be re-used to produce new polyisocyanurates or polyurethanes.

As consequence of the high chemical stability of polyisocyanurates, the development of chemical recycling methods so far focused on polyurethane foams comprising polyetherpolyols and having an isocyanate index of lower than 150.

Resource scarcity, climate change, environmental influences and an increasing awareness of sustainable products are leading to increased demands for new technologies to recycle plastics. Because of their large volumes in the market this applies in particular to polyurethanes but also to polyisocyanurates.

Therefore, a strong need exists to provide efficient and sustainable processes for recycling of polyisocyanurates to recover valuable raw materials in good yield and good quality, i.e. a quality allowing their re-use preferably as raw materials for new polyurethanes and polyisocyanurates.

Object of the present invention, therefore, was to provide a new process for depolymerization of polyisocyanurates as well as processes to isolate and recover the recycled hydrolysis products, preferably for re-use to produce chemicals.

A particular subject of the invention was to provide processes that can be carried out in standard equipment, i.e., steel reactors.

Another specific problem of the invention was to provide processes that can be operated at lower temperatures with good yields.

Another specific subject of the invention was to provide a process, which allows easy separation of the hydrolysis reactants, including optionally comprised phase transfer catalysts, from the products of the hydrolysis, i.e. the recovered raw materials that have been used to prepare the isocyanurate. The separation should be possible under mild conditions.

Another special subject of the invention was to provide a process with good yield and little side reactions. The process of the invention should allow that more than 85%, preferably more than 90%, particularly preferably more than 95% of the existing isocyanurate groups of the raw material are cleaved.

The process of the invention should be applicable to polyisocyanurates having a high isocyanate index, preferably of more than or equal to 150, preferably > 150 and more preferred > 250.

A further specific problem to be solved by the invention was to provide a process that allows to recover polyols and/or amines and/or polyamines in a quality very close to that of the raw materials used to produce the polyisocyanurates that were subjected to hydrolysis. It should be possible to use recovered polyols and/or amines and/or polyamines in high proportions for production of new chemicals, preferably isocyanates, polyurethanes, polyisocyanurates or polyureas or for re-use in other applications like as epoxy curing agents or as crosslinkers for other polymers.

Further problems not explicitly mentioned before can be derived from the overall content of the subsequent description, examples, and claims.

The inventors surprisingly found out, that a method of hydrolyzing a polyisocyanurate wherein
the polyisocyanurate is produced by reacting one or more polyol(s) selected from the group consisting of polyesterpolyol, mixture of polyesterpolyols and mixture of polyester- and polyetherpolyols, with an excess of one or more isocyanate(s) selected from the group consisting of organic isocyanate, mixture of organic isocyanates, organic polyisocyanate, mixture of organic polyisocyanates and mixture of organic isocyanate(s) and organic polyisocyanate(s),
   and wherein
the hydrolysis is carried out by contacting the polyisocyanurate with water in the presence of a base to yield:
   one or more carboxylic acid(s) comprising equal to or more than 2 carboxylic acid groups per molecule and corresponding to the carboxylic acid(s) used to prepare the polyesterpolyol(s) that was/were used to prepare the polyisocyanurate
      and
   one or more polyol(s) corresponding to the polyol(s) used to prepare the polyesterpolyol(s) that was/were used to prepare the polyisocyanurate,
      and
   one or more organic amine and/or polyamine, which correspond(s) to the organic isocyanate or polyisocyanate used to prepare the polyisocyanurate,
   and wherein
the base comprises one or more base(s) selected from the group consisting of
a base comprising an alkali metal cation and/or an ammonium cation and having a pK_{b} value at 25°C of from 1 to 10, which preferably does not comprise primary, secondary and or tertiary amino groups,
   and
a strong inorganic base having a pK_{b} value at 25°C of < 1,
   allows to recover the raw materials of the polyisocyanurate, i.e. the carboxylic acid(s) and/or the polyol(s) and/or the organic amine and/or the organic polyamine in high yields and purities.

The process of the invention provides a particularly high cleavage rate of the existing isocyanurate groups of more than 85%, preferably more than 90%, particularly preferably more than 95%.

In addition to cleavage of the existing isocyanurate groups, the process of the invention leads to cleavage of the polyesterpolyols that were used to prepare the polyisocyanurate, into its raw materials, i.e. the corresponding carboxylic acid(s) and polyols(s) can be recovered. These reaction products can easily be separated from the obtained amines formed as further reaction product. Thus, recovery of the recycled raw materials in high purity could be achieved.

The recycled amine component of the isocyanurate, preferably an aromatic amine component, can for example be recovered in a purity that enables phosgenation to polyisocyanates. The polyol(s) and the carboxylic acid(s) are also obtained in a purity that allows re-use for the production of polyurethanes or polyisocyanurates or use in other applications like polyesters. In particular fine-celled, uniform and low-interference foams can be produced, which meet all requirements in terms of e.g. density, strength or emissions. Advantageously the recycled products of the invention can be used to prepare new polyurethanes or polyisocyanurate foams without negatively affecting properties of the foam.

The method is particularly beneficial because it allows recycling of polyester polyol-based polyisocyanurate foams with a high isocyanate index of more than or equal to 150, preferably > 150 and more preferred > 250, which was not possible before.

A wide variety of cheap and/or low or non-corrosive bases can be used in the process of the invention to effectively depolymerize polyisocyanurates. In particular the base comprising an alkali metal cation and/or an ammonium cation and having a pK_{b} value at 25°C of from 1 to 10, which preferably do not comprise primary, secondary and or tertiary amino groups, is a weak inorganic bases, preferably weak, non-corrosive inorganic base. This allows that the process of the invention can be carried out in standard equipment under low or non-corrosive conditions.

In a preferred embodiment a phase transfer catalysts is added during hydrolysis, i.e. a base-catalyst combination is used. Use of such base-catalyst combination allows to improve the room time yield but increases the effort to separate and purify the reaction products. This is because the phase transfer catalyst is an additional component that needs to be separated from the reaction products. Use of a phase transfer catalyst also causes additional costs. Nevertheless, the improvement of room-time-yield may overcompensate the disadvantages of the use of a phase transfer catalyst. The process of the invention is very flexible and allows a man skilled in the art to optimize the process by either using the base only or by using the base-catalyst combination.

It is, thus, preferred that the method of the present invention comprises a method of hydrolyzing a polyisocyanurate wherein
the polyisocyanurate is produced by reacting one or more polyol(s) selected from the group consisting of polyesterpolyol, mixture of polyesterpolyols and mixture of polyester- and polyetherpolyols, with an excess of one or more isocyanate(s) selected from the group consisting of organic isocyanate, mixture of organic isocyanates, organic polyisocyanate, mixture of organic polyisocyanates and mixture of organic isocyanate(s) and organic polyisocyanate(s), wherein
the hydrolysis is carried out by contacting the polyisocyanurate with water in the presence of a base-catalyst-combination comprising the base and a catalyst, wherein the base-catalyst-combination is selected from the group consisting of base-catalyst-combinations (I), (II) or (III), to yield
   one or more carboxylic acid(s) comprising equal to or more than 2 carboxylic acid groups per molecule and corresponding to the carboxylic acid(s) used to prepare the polyesterpolyol(s) that was/were used to prepare the polyisocyanurate and
   one or more polyol(s) corresponding to the polyol(s) used to prepare the polyesterpolyol(s) that was/were used to prepare the polyisocyanurate, and
   one or more organic amine and/or polyamine, which correspond(s) to the organic isocyanate or polyisocyanate used to prepare the polyisocyanurate,
   wherein
the base-catalyst-combination (I) comprises the base comprising an alkali metal cation and/or an ammonium cation and having a pKb value at 25°C of from 1 to 10, which preferably does not comprise primary, secondary and or tertiary amino groups, and a catalyst selected from the group consisting of quaternary ammonium salts containing an ammonium cation containing 6 to 30 carbon atoms and organic sulfonates containing at least 7 carbon atoms, and wherein
the base-catalyst-combination (II) comprises the strong inorganic base having a pKb value at 25°C of < 1, and as catalyst a quaternary ammonium salt containing an ammonium cation containing 6 to 14 carbon atoms, preferably 6 to 12 carbon atoms if the ammonium cation comprises a benzyl residue, and wherein
the base-catalyst-combination (III) comprises the strong inorganic base having a pKb value at 25°C of < 1, and as catalyst a quaternary ammonium salt containing an ammonium cation containing 15 to 30 carbon atoms, preferably 15 to 28, more preferred 15 to 24, even more preferred 16 to 22 and most preferred 16 to 20, which method allows to recover the raw materials of the polyisocyanurate, i.e. the carboxylic acid(s) and/or the polyol(s) and/or the organic amine and/or the organic polyamine in high yields and purities.

The inventors found out, that it is preferred to use ammonium cations as phase transfer catalyst. Ammonium cations with a low number of carbon atoms, i.e. below 15, can be effectively used as well as such with a higher number of carbon atoms, i.e. 15 to 30. Use of such phase transfer catalysts allows to increase the yields and increases flexibility with regard to the reaction temperature.

Without being bond to any theory inventors believe, that the specific mild reaction conditions that can be applied in the process of the invention, especially in the process comprising use of the base-catalyst-combinations (I), (II) and (III), avoids the formation of by-products, which could cause problems during re-use of the recovered products.

Further benefits will be apparent from the subsequent description, examples, claims and figures.

### Detailed Description

Before describing the invention in more details, some important terms are defined as follows:
The verb "to comprise" as is used in the description, examples and the claims and its conjugation is used in its non-limiting sense to mean that items following the word are included, but items not specifically mentioned are not excluded. "Comprising" includes "consisting of" meaning that items following the word "comprising", are included without any additional, not specifically mentioned items, as preferred embodiment.

Reference to an element be the indefinite article "a" or "an" does not exclude the possibility that more than one of the elements is present, unless the context clearly requires that there is one and only one of the elements. The indefinite article "a" or "an" thus usually means "one or more".

The terms "catalyst" and "activating agent" are used synonymously in the present invention.

Polyurethane (PU) in the context of the present invention is especially understood to mean a product obtainable by reaction of polyisocyanates and polyols, or compounds having isocyanate-reactive groups. The polyurethanes which may be subjected to the process of the present invention are those prepared from active hydrogen-containing polyethers and polyisocyanates. Polyurethanes of this type are well known and are described, for example, in Ulrich, "Urethane Polymers", in Encyclopedia of Chemical Technology, Vol. 23, pp. 576-608(1983) and Backus et al., "Polyurethanes", in Encyclopedia of Polymer Science and Technology, Vol. 13, pp. 243-303(1988). Any known polyurethane can be used in the process of the invention, preferably the polyurethane is polyurethane waste.

Polyisocyanurate (PIR) in the context of the present invention is understood to mean a product obtainable by reaction of a polyol component with an excess of a polyisocyanate component, wherein the polyol component comprises polyester polyols or mixtures of polyester and polyether polyols. Preferably the isocyanate index of the PIR being 150 or more. During the reaction of the polyol component with an excess of the polyisocyanate component urethane structures are formed, arising as a result of the reaction of isocyanates with compounds having reactive hydrogen atoms of the polyol component, and, via reaction of the isocyanate groups with one another, there is formation of isocyanurate structures or further structures that result from the reaction of isocyanate groups with other groups, for example polyurethane groups. Polyisocyanurates have likewise long been known and described in the prior art.

The isocyanate index as used in the present invention is to be understood as the mole ratio of the polyisocyanate component to polyol component multiplied with 100 or in other words the mole ratio of isocyanate groups to isocyanate reactive groups multiplied with 100.

Preferably rigid PIR foam is used in the present. "Rigid foam" is a fixed technical term. The well-known and fundamental difference between soft foam and rigid foam is that a soft foam shows an elastic behavior and therefore the deformation is reversible. Rigid foam, on the other hand, is permanently deformed. Further information on rigid foams can also be found in the "Kunststoffhandbuch, Band 7, Polyurethane", Carl Hanser Verlag, 3rd edition 1993, chapter 6. The terms "hart foam" or "rigid foam" are handled synonymously in the sense of this invention.

The process of the invention is a method of hydrolyzing a polyisocyanurate, characterized in that
the polyisocyanurate is produced by reacting one or more polyol(s) selected from the group consisting of polyesterpolyol, mixture of polyesterpolyols and mixture of polyester- and polyetherpolyols with an excess of one or more isocyanate(s) selected from the group consisting of organic isocyanate, mixture of organic isocyanates, organic polyisocyanate, mixture of organic polyisocyanates and mixture of organic isocyanate(s) and organic polyisocyanate(s),
   and that
the hydrolysis is carried out by contacting the polyisocyanurate with water in the presence of a base or a base-catalyst-combination (I), (II) or (III) to yield:
   one or more carboxylic acid(s) comprising equal to or more than 2 carboxylic acid groups per molecule and corresponding to the carboxylic acid(s) used to prepare the polyesterpolyol(s) that was/were used to prepare the polyisocyanurate,
      and
   one or more polyol(s) corresponding to the polyol(s) used to prepare the polyesterpolyol(s) that was/were used to prepare the polyisocyanurate,
      and
   one or more organic amine and/or polyamine, which correspond(s) to the organic isocyanate or polyisocyanate used to prepare the polyisocyanurate,
   wherein
the base comprises one or more base(s) selected from the group consisting of
   a base comprising an alkali metal cation and/or an ammonium cation and having a pKb value at 25°C of from 1 to 10, which preferably does not comprise primary, secondary and or tertiary amino groups,
      and
   a strong inorganic base having a pKb value at 25°C of < 1,
   and wherein
the base-catalyst-combination (I) comprises the base comprising an alkali metal cation and/or an ammonium cation and having a pK_{b} value at 25°C of from 1 to 10, which preferably does not comprise primary, secondary and or tertiary amino groups, and a catalyst selected from the group consisting of quaternary ammonium salts containing an ammonium cation containing 6 to 30 carbon atoms and organic sulfonates containing at least 7 carbon atoms,
   and wherein
the base-catalyst-combination (II) comprises the strong inorganic base having a pK_{b} value at 25°C of < 1, and as catalyst a quaternary ammonium salt containing an ammonium cation containing 6 to 14 carbon atoms, preferably 6 to 12 carbon atoms if the ammonium cation comprises a benzyl residue,
   and wherein
the base-catalyst-combination (III) comprises the strong inorganic base having a pK_{b} value at 25°C of < 1, and as catalyst a quaternary ammonium salt containing an ammonium cation containing 15 to 30 carbon atoms, preferably 15 to 28, more preferred 15 to 24, even more preferred 16 to 22 and most preferred 16 to 20.

A preferred PIR, more preferred a PIR foam, even more preferred a rigid PIR foam, used in the process of the invention is produced by reacting
a) One or more polyol(s) selected from the group consisting of polyesterpolyol, mixture of polyesterpolyols, mixture of polyester- and polyetherpolyol, and comprising isocyanate-reactive groups selected from OH groups, SH groups, NH groups, NH₂ groups and mixtures thereof, preferably OH groups, with
b) an excess of one or more isocyanate(s) selected from the group consisting of organic isocyanate, mixture of organic isocyanates, organic polyisocyanate, mixture of organic polyisocyanates and mixture of organic isocyanate(s) and organic polyisocyanate(s), in the presence of
c) a catalyst catalyzing the reaction of the isocyanate reactive groups with the isocyanate groups and / or the reaction of isocyanate groups with each other, with the proviso that at least one trimerization catalyst is comprised
d) optionally a foam stabilizer
e) optionally a propellant
f) optionally further additives

Preferably the at least one polyol component a) comprises 2 or more isocyanate-reactive groups, selected from group consisting of OH groups, SH groups, NH groups, NH₂ groups and mixtures thereof, preferably OH groups. More preferred the polyol or the mixture of polyol(s) used as component a) having in sum on an average 1.8 to 8, preferably 1.9 to 5, more preferred 2 to 3 and most preferred 2 of said isocyanate-reactive groups and on an average 2 to 12, preferably 2 to 10, more preferred 2 to 6 carbon atoms. A functionality that is not an integer, e.g. 1.8, can result from the fact that at least one polyol with a higher functionality, e.g. greater than or equal to 2, is mixed with at least one polyol with a functionality of e.g. 1.

The polyol(s) may comprise ether and/or carbonate functional groups, preferably polyetherpolyol or polyethercarbonatpolyol.

Preferably polyester polyols based on esters of polybasic aliphatic or aromatic carboxylic acids, or a mixture of aromatic and aliphatic carboxylic acids, wherein the carboxylic acid(s) having 2 or 3, more preferred 2 carboxylic acid groups and having 2 to 12, preferably 4 to 10, more preferred 6 to 10 carbon atoms per molecule, are used as component a). Preferred aliphatic carboxylic acids are succinic acid, glutaric acid, adipic acid, suberic acid, azelaic acid, sebacic acid, decanedicarboxylic acid, maleic acid and fumaric acid and preferred aromatic carboxylic acids are phthalic acid, preferably (ortho)-phthalic acid, isophthalic acid, terephthalic acid and the isomeric naphthalenedicarboxylic acids. The polyester polyols are obtained by condensation of these polybasic carboxylic acids with polyols, i.e. polyhydric alcohols, preferably diols or triols having 2 to 12, more preferred 2 to 6, carbon atoms. Particular preferred are diols on the basis of glycol and/or glycol ether having a molecular weight below 180 g/mol, preferably below 140 g/mol, most preferred are monoethylene glycol and/or diethylene glycol. Preferably the polyester polyols used to produce PIR contain an excess of the polyhydric alcohols, so that they can also be present in unbound form in the polyol component. Particular preferred polyester polyols comprise a high content of aromatic monomers. PIR made from such polyester polyols exhibit very high flame retardancy.

In a preferred embodiment at least 90%, preferably at least 95%, more preferably at least 98% of the polyol components used to prepare the polyisocyanurate are polyesterpolyols having a hydroxyl number of 100 to 450 mg KOH / g, preferably 120 to 400 mg KOH / g, more preferably 140 to 350 mg KOH / g.

Beside of polyester polyols one or more polyol selected from the group consisting of polyether polyols, polyether polycarbonate polyols, natural oil-based polyols (NOPs; described in WO 2005/033167, US 2006/0293400, WO 2006/094227, WO 2004/096882, US 2002/0103091, WO 2006/116456, EP 1678232), filled polyols and prepolymer-based polyols, can be used in step a).

As isocyanates b) all isocyanates or polyisocyanates containing at least two isocyanate groups can be used. Suitable isocyanates and polyisocyanates for the purposes of this invention are all organic isocyanates having two or more isocyanate groups. It is generally possible to use aliphatic, cycloaliphatic, arylaliphatic with 2 or more, preferably 2 to 4 isocyanate groups and mixtures thereof. Such aromatic polyfunctional isocyanates are known per se. Preferably used are alkylene diisocyanates having 4 to 12 carbon atoms in the alkylene radical, e.g. dodecane 1,12-diisocyanate, 2-ethyltetramethylene 1,4-diisocyanate, 2-methylpentamethylene 1,5-diisocyanate, tetramethylene 1,4-diisocyanate, pentamethylene diisocyanate (PDI) and preferably hexamethylene 1,6-diisocyanate (HMDI), cycloaliphatic diisocyanates such as cyclohexane 1,3-and 1,4-diisocyanate and also any mixtures of these isomers, 4,4'-Methylenedicyclohexyldiisocyanat (H12MDI), 1-isocyanato-3,3,5-trimethyl-5-isocyanatomethylcyclohexane (isophorone diisocyanate or IPDI for short), hexahydrotolylene 2,4-and 2,6-diisocyanate and also the corresponding isomer mixtures, and preferably aromatic diisocyanates and polyisocyanates, for example toluene 2,4- and 2,6-diisocyanate (TDI) and the corresponding isomer mixtures, naphthalene diisocyanate, diethyltoluene diisocyanate, mixtures of diphenylmethane 4, 4', 2,4'- and 2,2'-diisocyanates (MDI) and polyphenylpolymethylene polyisocyanates (PMDI, also known as polymeric and crude MDI) and and their higher condensed analogues with an average functionality of 2 to 4. Further examples are mixtures of crude MDI and toluene diisocyanates (TDI). The organic diisocyanates and polyisocyanates may be used individually or in the form of mixtures thereof. It is likewise possible to use corresponding "oligomers" of the diisocyanates (IPDI trimer based on isocyanurate, biurets, uretdiones). In addition, the use of prepolymers based on the abovementioned isocyanates is possible. It is also possible to use isocyanates modified by the incorporation of urethane, uretdione, isocyanurate, allophanate and other groups, so-called modified isocyanates. Examples of particularly suitable isocyanates are also listed, for example, in EP 1712578, EP 1161474, WO 00/58383, US 2007/0072951, EP 1678232 and WO 2005/085310, which are entirely incorporated by reference.

Most preferred are organic polyisocyanates isomers of toluene diisocyanate (toluene 2,4- and 2,6-diisocyanate (TDI), in pure form or as isomer mixtures of varying composition), diphenylmethane 4,4'-diisocyanate (MDI), "crude MDI" or "polymeric MDI" (comprising the 4,4' isomer and also the 2,4' and 2,2' isomers of MDI and products having more than two rings) and also the two-ring product referred to as "pure MDI" that is composed predominantly of 2,4' and 4,4' isomer mixtures, and prepolymers derived therefrom. Examples of particularly suitable isocyanates are detailed, for example, in EP 1712578, EP 1161474, WO 00/58383, US 2007/0072951, EP 1678232 and WO 2005/085310, which are hereby fully incorporated by reference.

As catalyst(s) c) are all compounds capable of accelerating the reaction of isocyanates with OH functions, NH functions or other isocyanate-reactive groups and with isocyanates themselves can be used. It is possible here with preference to make use of customary catalysts known in the art, including, for example, amines (cyclic, acyclic; monoamines, diamines, oligomers having one or more amino groups), ammonium compounds, organometallic compounds and metal salts, preferably those of potassium, tin, iron, zinc or bismuth. It is also preferred to use mixtures of more than one of these catalysts.

Foam stabilizers (d) and their use in the production of PIR foams are known to those skilled in the art. As foam stabilizers, in particular, surface-active compounds (surfactants) can be used. Even though the use of foam stabilizers is optional, they are preferably used in the production of PIR foams. They can be used to optimize the desired cell structure and the foaming process. These compounds are swell known in the art. Siloxanes are described, for example, in the following patent specifications, but the use is only described in classic PU foams (e.g. as molded foam, mattress, insulation material, building foam, etc.): CN 103665385, CN 103657518, CN 103055759, CN 103044687, US 2008/0125503, US 2015/0057384, EP 1520870 A1, EP 1211279, EP 0867464, EP 0867465, EP 0275563. In addition to surface-active Si-containing compounds, Si-free surfactants can also be used. For example, in EP 2295485 A1 the use of lecithin and in US 3746663 the use of vinylpyrrolidone-based structures as a foam stabilizer, but only for the production of PU rigid foam, is described. Further Si-free foam stabilizers are described, for example, in EP 2511328 B1, DE 1020011007479 A1, DE 3724716 C1, EP 0734404, EP 1985642, DE 2244350 and US 5236961.

The use of blowing agents to produce PIR foams is also well known in the art. It is possible to work with chemical and physical blowing agents. The choice of blowing agent strongly dependents on the nature of the system. Depending on the amount of blowing agent used, a foam with high or low density is produced. Thus, foams with densities of 5 kg / m³ to 900 kg / m3, preferably 5 to 350, more preferably 8 to 200 kg / m3, in particular 8 to 150 kg / m³ can be prepared.

Preferred physical blowing agents used may be corresponding compounds having appropriate boiling points. Examples of blowing agents are liquefied CO₂, nitrogen, air, volatile liquids, for example hydrocarbons having 3, 4 or 5 carbon atoms, preferably cyclo-, iso- and n-pentane, hydrofluorocarbons, preferably HFC 245fa, HFC 134a and HFC 365mfc, hydrochlorofluorocarbons, preferably HCFC 141b, hydrofluoroolefins (HFOs) or hydrohaloolefins such as for example 1234ze, 1234yf, 1233zd(E) or 1336mzz, oxygen-containing compounds such as methyl formate, cetones, preferably acetone, ethers, preferably dimethoxymethane, or chlorinated hydrocarbons, preferably dichloromethane and 1,2-dichloroethane.

It is likewise optionally possible to use chemical blowing agents which react with NCO groups to liberate gases, for example water or formic acid. As chemical blowing agents, one or more compounds can be used that react with NCO groups by releasing gases, such as water or formic acid, or by the temperature rise during the reaction release gases such as sodium bicarbonate. Optional additives f) that may be comprised in PIR include all substances which are known from the prior art and are used in the production of polyisocyanurates, especially polyisocyanurate foams, for example crosslinkers and chain extenders, stabilizers against oxidative degradation (known as antioxidants), flame retardants, surfactants, biocides, cell-refining additives, cell openers, solid fillers, antistatic additives, nucleating agents, thickeners, dyes, pigments, colour pastes, fragrances, and emulsifiers, etc.

The process for producing rigid PIR foams can be conducted by known methods, for example by manual mixing or preferably by means of foaming machines. If the process is carried out by using foaming machines, it is possible to use high-pressure or low-pressure machines.

The polyisocyanurate used in the process of the invention preferably has an isocyanate index of equal to or more than 150, preferably >180, more preferred > 250, most preferred > 250 to 500. Such polyisocyanates are particular stable and having a very high chemical resistance and/or flame retardancy. They, thus, show the highest market potential for the inventive PIR recycling process.

In a first particular preferred embodiment of the invention the hydrolysis is carried out by contacting the PIR with water in the presence of a base without use of a phase transfer catalyst.

Preferably the base used in this first particular preferred embodiment comprises, more preferred consists of, one or more bases selected from the group consisting of
a base comprising an alkali metal cation and/or an ammonium cation and having a pK_{b} value at 25°C of from 1 to 10 preferably 1 to 8, more preferred 1 to 7 and most preferred 1.5 to 6, which preferably does not comprise primary, secondary and or tertiary amino groups,
   and
a strong inorganic base having a pK_{b} value at 25°C of < 1.

More preferred bases comprising an alkali metal cation and/or an ammonium cation and having a pK_{b} value at 25°C of from 1 to 10, are weak, low or non-corrosive bases. Also preferably said bases do not comprise primary, secondary and or tertiary amino groups. Particularly preferred the base used in the first particular preferred embodiment is selected from the group consisting of alkali metal phosphates, alkali metal hydrogen phosphates, alkali metal carbonates, alkali metal silicates, alkali metal hydrogen carbonates, alkali metal acetates, alkali metal sulfites, ammonium hydroxide, and mixtures thereof. Even more preferred the base is selected from the group consisting of alkali metal phosphates, alkali metal carbonates, alkali metal silicates, ammonium hydroxide, and mixtures thereof. Most preferred the base is selected from the group consisting of alkali metal carbonates, alkali metal silicates, and mixtures thereof.

Ammonium cation in the base used in the first particular preferred embodiment includes NH4⁺, NHR₃⁺, NH₂R₂⁺, NH₃R⁺, for example ammonium hydroxide includes NH₄OH, NHR₃OH, NH₂R₂OH, NH₃ROH, wherein R stand for an organic residue and wherein the residues R in the ammonium cations may be identical or different. Preferably ammonium cation of the base stands for NH₄⁺. Particular preferred the base does not comprise alkaline earth metal cations because of the limited solubility of such bases in water.

Preferred alkali metals are selected from the group consisting of Na, K and Li and mixtures thereof, most preferred Na and K and mixtures thereof.

Preferably the weak base is used in this first particular preferred embodiment in form of a base solution comprising the base and water, even more preferred as a saturated base solution. If a saturated base solution is used it is preferred that the weight ratio of saturated base solution to polyisocyanurate, calculated at 25°C, is in the range of from of 0.5 to 25, more preferred 0.5 to 15, even more preferred 1 to 10 and most preferred 2 to 7.

Use of the bases described before, i.e. bases comprising an alkali metal cation and/or an ammonium cation and having a pK_{b} value at 25°C of from 1 to 10, which preferably does not comprise primary, secondary and or tertiary amino groups, allows to run the process of the invention in standard equipment, preferably in steel reactors, without special corrosion protection and thus, significantly contributes to a reduction of the invest costs for the plants. It is also possible to use very cheap bases that contribute to reduced operating costs.

If the strong inorganic base(s) is/are used, it is preferred that the strong inorganic base(s) having a pK_{b} value at 25°C of < 1, more preferably 0.5 to -4, even more preferred 0.25 to -3.5 and most preferred 0 to -2.9. It is further preferred that the strong inorganic bases do not comprise CH bonds and/or primary, secondary and or tertiary amino groups.

Particular preferred the strong inorganic base is selected from the group consisting of alkali metal hydroxides, alkali metal oxides, alkaline earth metal hydroxides, alkaline earth metal oxides and mixtures thereof. Preferred alkali metals are selected from the group consisting of Na, K and Li and mixtures thereof, most preferred Na and K and mixtures thereof. Preferred alkaline earth metals are selected from the group consisting of Be, Mg, Ca, Sr, Ba and mixtures thereof, most preferred Mg and Ca and mixtures thereof.

Use of strong inorganic bases as described before allows to run the process of the invention with very high yields at lower temperatures and thus, significantly contributes to a reduction of the operating costs.

The strong inorganic base is preferably used in form of a base solution comprising a strong inorganic base and water. For an efficient conversion rate, it is particular preferred if the concentration of base in the base solution is higher than or equal to 5 weight percent, based on the weight of the base solution, preferably 5 to 70 weight percent, more preferred 5 to 60 weight percent, even more preferred 10 to 50 weight percent, particular preferred 15 to 40 weight percent and most preferred 20 to 40 weight percent.

The amount of the base in the reaction mixture must be sufficient to catalyze the desired hydrolysis of the PIR at a practicable rate. Preferably the weight ratio of base to PIR is from 0.01 to 25, more preferred 0.1 to 15, even more preferred 0.2 to 10 and most preferred 0.5 to 5.

In a second particular preferred embodiment the hydrolysis is carried out by contacting the PIR with water in the presence of the base-catalyst-combination (I), wherein the base comprises an alkali metal cation and/or an ammonium cation and has a pK_{b} value at 25°C of from 1 to 10, and the catalyst is selected from the group consisting of quaternary ammonium salts containing an ammonium cation containing 6 to 30 carbon atoms and organic sulfonates containing at least 7 carbon atoms.

The base used in this second particular preferred embodiment comprises an alkali metal cation and/or an ammonium cation and has a pK_{b} value at 25°C of from 1 to 10, preferably 1 to 8, more preferred 1 to 7 and most preferred 1.5 to 6. Preferably low or non-corrosive bases are used. Also preferably the base does not comprise primary, secondary and or tertiary amino groups. Particular preferred, the base is selected from the group consisting of alkali metal phosphates, alkali metal hydrogen phosphates, alkali metal carbonates, alkali metal silicates. alkali metal hydrogen carbonates, alkali metal acetates, alkali metal sulfites, ammonium hydroxide, and mixtures thereof. Even more preferred a base is selected from the group consisting of alkali metal phosphates, alkali metal carbonates, alkali metal silicates, ammonium hydroxide, and mixtures thereof. Most preferred the base is selected from the group consisting of alkali metal carbonates, alkali metal silicates, and mixtures thereof.

Ammonium cation in the base used in this second particular preferred embodiment of the invention includes NH₄⁺, NHR₃⁺, NH₂R₂⁺, NH₃R⁺, for example ammonium hydroxide includes NH₄OH, NHR₃OH, NH₂R₂OH, NH₃ROH, wherein R stand for an organic residue and wherein the residues R in the ammonium cations may be identical or different. Preferably ammonium cation of the base stands for NH₄⁺. Particular preferred the base of the invention does not comprise alkaline earth metal cations because of the limited solubility of such bases in water.

Preferred alkali metals are selected from the group consisting of Na, K and Li and mixtures thereof, most preferred Na and K and mixtures thereof.

Use of the bases described before allows to run the process of the invention in standard equipment, preferably in steel reactors, without special corrosion protection and thus, significantly contributes to a reduction of the invest costs for the plants. It is also possible to use very cheap bases that contribute to reduced operating costs.

The amount of the base in the reaction mixture of this second particular preferred embodiment must be sufficient to catalyze the desired hydrolysis of the PIR at a practicable rate. Preferably the weight ratio base to PIR is in the range of from 0.01 to 50, more preferred 0.1 to 25 and most preferred 0.5 to 20. Preferably the base is used in form of a base solution comprising a base and water, even more preferred as a saturated base solution. If a saturated base solution is used it is preferred that the weight ratio of saturated base solution to polyisocyanurate, calculated at 25°C, is in the range of from of 0.5 to 25, more preferred 0.5 to 15, even more preferred 1 to 10 and most preferred 2 to 7.

In a third particular preferred embodiment the hydrolysis is carried out by contacting PIR with water in the presence of a base-catalyst-combination (II), wherein the base is a strong inorganic base having a pK_{b} value at 25°C of < 1, and the catalyst is a quaternary ammonium salt containing an ammonium cation containing 6 to 14 carbon atoms, preferably 6 to 12 carbon atoms if the ammonium cation comprises a benzyl residue.

The strong inorganic base used in this third particular preferred embodiment preferably is a strong inorganic base having a pK_{b} value at 25°C of below 1, preferably 0.5 to -4, more preferred 0.25 to - 3.5 and most preferred 0 to -2.9. Preferred strong inorganic bases are bases that do not comprise CH bonds and/or primary, secondary and or tertiary amino groups.

Particular preferred the strong inorganic base used in this third particular preferred embodiment is selected from the group consisting of alkali metal hydroxides, alkali metal oxides, alkaline earth metal hydroxides, alkaline earth metal oxides and mixtures thereof. Preferred alkali metals are selected from the group consisting of Na, K and Li and mixtures thereof, most preferred Na and K and mixtures thereof. Preferred alkaline earth metals are selected from the group consisting of Be, Mg, Ca, Sr, Ba and mixtures thereof, most preferred Mg and Ca and mixtures thereof. Most preferred alkali metals selected from the group consisting of potassium or sodium and mixtures thereof are used.

Use of the bases described before allows to run the process of the invention with very higher yields at lower temperatures and thus, significantly contributes to a reduction of the operating costs.

The amount of base in the reaction mixture must be sufficient to catalyze the desired hydrolysis of the PIR at a practicable rate. Preferably the weight ratio of base to PIR is from 0.01 to 25, more preferred 0.1 to 15, even more preferred 0.2 to 10 and most preferred 0.5 to 5. The base is preferably used in form of a base solution comprising a base and water. For an efficient conversion rate, it is particular preferred if the concentration of base in the base solution is higher than or equal to 5 weight %, based on the weight of the base solution, preferably 5 to 70 weight percent, more preferred 5 to 60 weight percent, even more preferred 10 to 50 weight percent, particular preferred 15 to 40 weight percent and most preferred 20 to 40 weight percent.

In a fourth particular preferred embodiment the hydrolysis is carried out by contacting PIR with water in the presence of a base-catalyst-combination (III), wherein the base is a strong inorganic base having a pK_{b} value at 25°C of < 1, and the catalyst is a quaternary ammonium salt containing an ammonium cation containing 15 to 30 carbon atoms, preferably 15 to 28, more preferred 15 to 24, even more preferred 16 to 22 and most preferred 16 to 20.

The strong inorganic base in base-catalyst-combination (III) preferably is a strong inorganic base having a pK_{b} value at 25°C of below 1, preferably 0.5 to -4, more preferred 0.25 to -3.5 and most preferred 0 to -2.9. Preferred strong inorganic bases are bases that do not comprise CH bonds and/or primary, secondary and or tertiary amino groups.

More preferred the strong inorganic base is selected from the group consisting of alkali metal hydroxides, alkali metal oxides, alkaline earth metal hydroxides, alkaline earth metal oxides and mixtures thereof. Even more preferred the alkali metals of the base are selected from the group consisting of Na, K and Li and mixtures thereof, most preferred Na and K and mixtures thereof and/or the alkaline earth metals are selected from the group consisting of Be, Mg, Ca, Sr, Ba and mixtures thereof, preferably Mg and Ca and mixtures thereof. Most preferred alkali metals selected from the group consisting of potassium or sodium and mixtures thereof are used.

Use of the bases described before in this fourth particular preferred embodiment allows to run the process of the invention with very higher yields at lower temperatures and thus, significantly contributes to a reduction of the operating costs.

The amount of base in the reaction mixture in this fourth particular preferred embodiment must be sufficient to catalyze the desired hydrolysis of the PIR at a practicable rate. Preferably the weight ratio of base to PIR is from 0.01 to 25, more preferred 0.1 to 15, even more preferred 0.2 to 10 and most preferred 0.5 to 5. The base is preferably used in form of a base solution comprising a base and water. For an efficient conversion rate, it is particular preferred if the concentration of base in the base solution is higher than or equal to 5 weight percent, based on the weight of the base solution, preferably 5 to 70 weight percent, more preferred 5 to 60 weight percent, even more preferred 10 to 50 weight percent, particular preferred 15 to 40 weight percent and most preferred 20 to 40 weight percent.

It is preferred that the base or the base-catalyst-combinations (I), (II) and (III) or the reaction mixture during hydrolysis do not comprise non-ionic organic amin bases. "Non-ionic" means that the base is not in the form of a salt before being added to the reaction mixture, i.e. does not comprise an anion and a cation. "Organic amine bases" are compounds which, in addition to carbon and hydrogen contain nitrogen and react to salt-like compounds with acids. Preferably the "organic amine bases" comprise one or more CH bonds. Non-ionic organic amin bases would be obtained in the same phase as the amines that are obtained as reaction products during hydrolysis during and would cause additional efforts during separation of the amines that are obtained as reaction products.

The quaternary ammonium salt used as phase transfer catalyst in the base-catalyst-combinations (I), (II) and (III) preferably has the general structure R₁ R₂ R₃ R₄ NX wherein R₁,R₂,R₃, and R₄ are the same or different and are hydrocarbyl groups selected from alkyl, aryl, and arylalkyl and X is selected from the group consisting of halide, preferably chloride and/or bromide, hydrogen sulfate, alkyl sulfate, preferably methylsulfate and ethylsulfate, carbonate, hydrogen carbonate, carboxylate, preferably acetate, or hydroxide.

In the first particular preferred embodiment with the base-catalyst-combination (I) R₁, R₂, R₃, and R₄ and X are defined as follows:
- R₁ and R₂ are the same or different and are alkyl groups with 1 to 12, preferably 1 to 10, more preferred 1 to 7, even more preferred 1 to 6, especially preferred 1 to 5 and most preferred 1 to 4 carbon atoms, wherein the alkyl groups may be linear, branched, cyclic, saturated or unsaturated, most preferred are linear, saturated alkyl groups,
- R₃ is selected from the group consisting of alkyl groups with 1 to 12, preferably 1 to 10, more preferred 1 to 7, even more preferred 1 to 6, especially preferred 1 to 5 and most preferred 1 to 4 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and,
- R₄ is selected from the group consisting of alkyl groups with 3 to 12, preferably 3 to 10, more preferred 3 to 7, most preferred 4 to 6 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and saturated, and
- X is selected from the group consisting of halide, preferably chloride and/or bromide, hydrogen sulfate, alkyl sulfate, preferably methylsulfate and ethylsulfate, carbonate, hydrogen carbonate, acetate or hydroxide.

Even more preferred R₁ to R₄ are selected from the definitions provided above such that
the sum of carbon atoms in the quaternary ammonium cation is 6 to 14, preferably 7 to 14, more preferred 8 to 13
   or
that the sum of carbon atoms in the quaternary ammonium cation is 15 to 30, preferably 15 to 28, more preferred 15 to 24, even more preferred 16 to 22 and most preferred 16 to 20.

In the second particular preferred embodiment with the base-catalyst-combination (II) R₁, R₂, R₃, and R₄ and X are defined as follows:
- R₁ to R₃ are the same or different and are alkyl groups with 1 to 6, preferably 1 to 5, more preferred 1 to 4, even more preferred 1 to 3, especially preferred 1 or 2 and most preferred 1 carbon atoms, wherein the alkyl groups may be linear, branched, cyclic, saturated or unsaturated, most preferred are linear, saturated alkyl groups,
- R₄ is selected from the group consisting of alkyl groups with 3 to 11, preferably 3 to 10, more preferred 3 to 8, most preferred 4 to 6 carbon atoms, aryl groups with 6 to 11, preferably 6 to 10, and most preferred 6 to 8 carbon atoms, and aralkyl groups with 7 to 11, preferably 7 to 10, and most preferred 7 to 9 carbon atoms, wherein the alkyl groups may be linear, branched, cyclic, saturated or unsaturated, most preferred are linear, saturated alkyl groups, and
- X is selected from the group consisting of halide, preferably chloride and/or bromide, hydrogen sulfate, alkyl sulfate, preferably methylsulfate and ethylsulfate, carbonate, hydrogen carbonate, acetate or hydroxide.

Even more preferred R₁ to R₄ are selected from the definitions provided above such that
if R₄ is different from a benzyl residue, R₁ to R₄ are selected such that the sum of carbon atoms in the quaternary ammonium cation is 6 to 14, preferably 7 to 14, more preferred 8 to 13
   or
if R₄ is a benzyl residue, R₁ to R₃ are selected such that the sum of carbon atoms in the quaternary ammonium cation is 6 to 12, preferably 7 to 12, more preferred 8 to 11.

In the third particular preferred embodiment with the base-catalyst-combination (III) R₁, R₂, R₃, and R₄ and X are defined as follows:
- R₁ and R₂ are the same or different and are alkyl groups with 1 to 12, preferably 1 to 10, more preferred 1 to 7, even more preferred 1 to 6, especially preferred 1 to 5 and most preferred 1 to 4 carbon atoms, wherein the alkyl groups may be linear, branched, cyclic, saturated or unsaturated, most preferred are linear, saturated alkyl groups,
- R₃ is selected from the group consisting of alkyl groups with 1 to 12, preferably 1 to 10, more preferred 1 to 7, even more preferred 1 to 6, especially preferred 1 to 5 and most preferred 1 to 4 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and,

- R₄ is selected from the group consisting of alkyl groups with 3 to 12, preferably 3 to 10, more preferred 3 to 7, most preferred 4 to 6 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and saturated, and
- X is selected from the group consisting of halide, preferably chloride and/or bromide, hydrogen sulfate, alkyl sulfate, preferably methylsulfate and ethylsulfate, carbonate, hydrogen carbonate, acetate or hydroxide.

Even more preferred R₁ to R₄ are selected from the definitions provided above such that that the sum of carbon atoms in the quaternary ammonium cation is 15 to 30, preferably 15 to 28, more preferred 15 to 24, even more preferred 16 to 22 and most preferred 16 to 20.

If quaternary ammonium salts are used as phase transfer catalysts, although the addition of even trace amounts of these phase transfer catalysts will accelerate the hydrolysis rate, it is preferred that at least 0.5 weight percent phase transfer catalyst, based on the weight of the polyisocyanurate be used, more preferably 0.5 to 15 weight percent, even more preferred 1 to 10 weight percent, particular preferred more 1 to 8 weight percent, especially preferred 1 to 7 and most preferred 2 to 6 weight percent.

Water functions as a reactant in the inventive hydrolysis reaction and thus does not need to be present in stoichiometric excess relative to the isocyanurate functional groups in the polymer to be hydrolyzed, it will generally be desirable to utilize a substantial quantity of water in order that it may conveniently serve as a reaction medium and solvent or carrier for the strong base and activating agent. For these reasons, the water is preferably present in condensed (liquid) form. Typically, the weight ratio of PIR to water is from 3:1 to 1:15.

Preferably the polyisocyanurate is contacted with water and the base or with water, the base and the phase transfer catalyst,
at a temperature of from 90°C to 220°C, preferably 100°C to 210°C, more preferred 110°C to 200°C and most preferred 120°C to 190°C
   and/or
for 30 minutes to 20 hours, preferably 30 minutes to 16 hours, more preferred 30 minutes to 14 hours, even more preferred 45 minutes to 10 hours, particular preferred 60 minutes to 8 hours, especially preferred 60 minutes to 6 hours
   and/or
from 1 to 30 bara, preferably 2 to 20 bara, more preferred 3 to 15 bara.

These reaction conditions provide economic benefits in terms of energy consumption and space time yield. If the temperatures are too low conversion might be incomplete or reaction time will become too long. If the rection temperature is too high or the reaction time is expanded outside of the ranges given before, increased formation of side-products has been observed and energy consumption increased to an inacceptable level. The formation of side products can lead to unwanted coloration of the recovered products, which causes the need for additional purification steps. Conducting the reaction under elevated pressure has been found to shorten reaction time and allows to operate at lower temperatures.

To facilitate handling of the PIR, preferably PIR foam, it is preferably desirable to chop, pulverize, grind, or otherwise comminute the PIR such that it is in the form of relatively small particles or granules. If the PIR is a foam, it may be partially or fully compressed prior to contacting with the water and the organic amine base. If the PIR is in solid form, an initial pulverization step is highly advantageous so as to maximize the surface area available for reaction (thereby reducing the reaction time required to achieve the desired level of hydrolysis).

The process of this invention will result in the effective hydrolytic cleavage of the isocyanurate bonds present in the PIR being treated. Under the reaction conditions, the polyester polyols obtained after cleavage of the isocyanurate bonds are further hydrolysed to obtain the correlating carboxylic acids as well as polyols.

In the process of the invention, thus,
one or more carboxylic acid(s) comprising equal to or more than 2 carboxylic acid groups per molecule and corresponding to the carboxylic acid(s) used to prepare the polyesterpolyol(s) that was/were used to prepare the polyisocyanurate,
   and
one or more polyol(s) corresponding to the polyol(s) used to prepare the polyesterpolyol(s) that was/were used to prepare the polyisocyanurate,
   and
one or more organic amine and/or polyamine, which correspond(s) to the organic isocyanate or polyisocyanate used to prepare the polyisocyanurate
are obtained.

Preferably the one or more carboxylic acid(s) obtained after the hydrolysis is selected from the group consisting of phthalic acid, preferably (ortho)-phthalic acid, terephthalic acid, Isophthalic acid, and isomeric naphthalene dicarboxylic acids, succinic acid, glutaric acid, adipic acid, suberic acid, azelaic acid, sebacic acid, decandicarboxylic acid, maleic acid, fumaric acid and mixtures thereof.

Also preferred the one or more polyol(s) obtained after the hydrolysis is a diol on the basis of glycol and/or glycol ether having a molecular weight below 180 g/mol, preferably below 140 g/mol, particular preferred monoethylene glycol and/or diethylene glycol.

Further preferred the one or more organic amine and/or polyamine obtained after the hydrolysis is selected from the group consisting of dodecane 1,12-diamine, 2-ethyltetramethylene 1,4-diamine, 2-methylpentamethylene 1,5-diamine, tetramethylene 1,4-diamine, pentamethylene diamine (PDA) and preferably hexamethylene 1,6-diamine (HMDA), cycloaliphatic diamines such as cyclohexane 1,3- and 1,4-diamine and also any mixtures of these isomers, 4,4'-Methylenedicyclohexyldiisocyanat (H12MDI), 1-isocyanato-3,3,5-trimethyl-5-isocyanatomethylcyclohexane (isophorone diamine or IPDA for short), hexahydrotolylene 2,4- and 2,6-diamine and also the corresponding isomer mixtures, and preferably aromatic diamines and polyisocyanates, for example toluene 2,4- and 2,6-diamine (TDA) and the corresponding isomer mixtures, naphthalene diamine, diethyltoluene diamine, mixtures of diphenylmethane 4, 4', 2,4'-and 2,2'-diamines (MDA) and polyphenylpolymethylene polyamines and their higher condensed analogues with an average functionality of 2 to 4.

The hydrolysis reaction may be carried out as a batch, continuous, or semi-continuous process in any appropriate vessel or other apparatus (for example, a stirred tank reactor or screw extruder). It will generally be preferred to agitate or stir the reaction components so as to assure intimate contact, rapid hydrolysis rates, and adequate temperature control.

After completion of the hydrolysis step, it is preferred that the reaction products are separated from each other and optionally subjected to purification steps. Preferred separation and purification methods are selected from the group consisting of filtration, membrane separation, phase separation, chromatographic methods, distillation, extraction and combinations of said methods. Preferably the amine components obtained as reaction product of the hydrolysis are separated from the other components via distillation or extraction, more preferred via distillation.

The recovered products of the process of the invention, in particular the one or more carboxylic acid(s) and/or polyol(s) and/or organic amine(s) and/or polyamine(s), can be re-used for production of new chemical, preferably polyurethane, preferably polyurethane foam, or polyisocyanurates, preferably polyisocyanurate foams. The inventors found out that they can be used to produce polyurethane foams of PIR of high quality even without addition or with only minor addition of virgin polyol. This is a significant achievement.

The recovered amines can be converted to organic polyisocyanates by conventional processes and similarly employed as components of polyurethanes or PIR.

Without further elaboration, it is believed that one skilled in the art can, using the preceding description, utilize the present invention of its fullest extent. The following examples, therefore, are to be considered as merely illustrative and not limitative of the claims or remainder of the disclosure in any.

### Examples

### Preparation of PIR foam to be recycled

For the production of the PIR-foams, which were used for the hydrolysis trials described in the following Examples 1, 2 and 4, the formulation described in Table 1, respectively for Example 3, the formulation described in Table 2, were used. The PIR-foams were produced by manual mixing. For this purpose, polyol, flame retardant, catalysts, water, foam stabilizer, blowing agent were weighed into a beaker and mixed by means of a disc stirrer (6 cm in diameter) at 1000 rpm for 30 s. The blowing agent quantity which had evaporated during the mixing operation was determined by reweighing and replenished. Subsequently, the isocyanate (MDI) was added, and the reaction mixture was stirred with the stirrer described at 3000 rpm for 5 s. In the case of pour-in-place foaming, foaming was effected in the beaker itself; otherwise, the mixture was transferred into a paper-lined box of base area 27 x 14 cm.

**Table 1: PIR formulation for Examples 1, 2 and 4**

| **Component** | **Proportion by weight** |
|---|---|
| Polyesterpolyol* | 100 |
| Amine catalyst** | 0,5 |
| Trimerization catalyst*** | 3,5 |
| Foam stabilizer**** | 2 |
| Water | 0,6 |
| Flame retardant***** | 15 |
| Cyclopentane/lsopentane 70:30 | 8 |
| MDI****** | 263 |

| | |
|---|---|
| *Stepanpol^{®} PS 3152 from Stepan, OH-Zahl 315 mg KOH/g **POLYCAT^{®} 5 (Pentamethyldiethylenetriamine) from Evonik Operations GmbH ***KOSMOS^{®} 70 LO from Evonik Operations GmbH ****TEGOSTAB^{®} B 8411 from Evonik Operations GmbH *****Fyrol^{®} PCF from ICL Industrial Products ******Polymeric MDI, 200 mPa*s, 31,5% NCO, Functionality 2,7. | |

**Table 2: PIR formulation for Example 3**

| **Component** | **Proportion by weight** |
|---|---|
| Diethylene gylcol (DEG) | 13 |
| Amine catalyst* | 0,5 |
| Trimerization catalyst** | 3,4 |
| Foam stabilizer*** | 2 |
| Water | 0,6 |
| Cyclopentane/lsopentane 70:30 | 11 |
| MDI**** | 357 |

| | |
|---|---|
| *POLYCAT^{®} 5 from Evonik Operations GmbH **KOSMOS^{®} 70 LO from Evonik Operations GmbH ***TEGOSTAB^{®} B 8411 from Evonik Operations GmbH ****Polymeric MDI, 200 mPa*s, 31,5% NCO, Functionality 2,7. | |

### Example 1

PIR foam that was prepared as described above was grinded. 200 g of grounded PIR-foam were blended with 2083 g of an aqueous K₂CO₃-solution (w (K₂CO₃) = 40 %) and 12.5 g Tetra-n-butylammonium hydrogen sulfate. The formed suspension was transferred to a 5 L-pressure reactor. The mixture was heated to 170 °C and stirred for 5 h. A pressure of about 7 bar was built up. 2096 g of a two-phase product was obtained. The mixture was completely liquid, and no solid components could be seen.

The upper phase (130 g) was brown and consists of 75 - 80% MDA according to H-NMR studies. The C-NMR shows that only traces of urethane-, urea- and isocyanurate-groups are present and that the conversion rate is > 98 %.

### Example 2

PIR foam that was prepared as described above was grinded. 70 g of grounded PIR-foam were blended with 700 g of an aqueous NaOH-solution (w (NaOH) = 20 %) and 7 g Tetra-n-butylammonium hydrogen sulfate. The formed suspension was transferred to a 2 L-pressure reactor with PTFE-inliner. The mixture was heated to 150 °C and stirred for 14 h. A two-phase product was obtained. The upper oily layer (38 g) was separated. The mixture was completely liquid, and no solid components could be seen.

The oily phase was brown and consists of 75 - 80% MDA according to H-NMR studies.

The C-NMR shows that only traces of urethane-, urea- and isocyanurate-groups are present and that the conversion rate is > 98 %

### Example 3

PIR foam that was prepared as described above was grinded. 70 g of grounded PIR-form were blended with 700 g of an aqueous NaOH-solution (w (NaOH) = 20 %) and 7 g Tetra-n-butylammonium hydrogen sulfate. The formed suspension was transferred to a 2 L-pressure reactor with PTFE-inliner. The mixture was heated to 150 °C and stirred for 14 h. A two-phase product was obtained. The upper oily layer (38 g) was separated. The mixture was completely liquid, and no solid components could be seen.

The oily phase was brown and consists of 75 - 80% MDA according to H-NMR studies.

The C-NMR shows that only traces of urethane-, urea- and isocyanurate-groups are present and that the conversion rate is > 98 %.

### Example 4

PIR foam that was prepared as described above was grinded. 200 g of grounded PIR-foam were blended with 2080 g of an aqueous K₂CO₃-solution (w (K₂CO₃) = 40 %). The formed suspension was transferred to a 5 L-pressure reactor. The mixture was heated to 170 °C and stirred for 5 h. A pressure of about 7 bar was built up. 2080 g of a two-phase product was obtained. The mixture was completely liquid, and no solid components could be seen.

The upper phase (130 g) was brown and consists of > 80% MDA according to H-NMR studies. The C-NMR shows that only traces of urethane-, urea- and isocyanurate-groups are present and that the conversion rate is > 97 %.

Example 4 shows that the process of the invention can also be operated without phase transfer catalyst in good yield.

## Claims

1. A method of hydrolyzing a polyisocyanurate
**characterized in that**
the polyisocyanurate is produced by reacting one or more polyol(s) selected from the group consisting of polyesterpolyol, mixture of polyesterpolyols and mixture of polyester- and polyetherpolyols with an excess of one or more isocyanate(s) selected from the group consisting of organic isocyanate, mixture of organic isocyanates, organic polyisocyanate, mixture of organic polyisocyanates and mixture of organic isocyanate(s) and organic polyisocyanate(s),
and that
the hydrolysis is carried out by contacting the polyisocyanurate with water in the presence of a base to yield,
one or more carboxylic acid(s) comprising equal to or more than 2 carboxylic acid groups per molecule and corresponding to the carboxylic acid(s) used to prepare the polyesterpolyol(s) that was/were used to prepare the polyisocyanurate
and
one or more polyol(s) corresponding to the polyol(s) used to prepare the polyesterpolyol(s) that was/were used to prepare the polyisocyanurate,
and
one or more organic amine and/or polyamine, which correspond(s) to the organic isocyanate or polyisocyanate used to prepare the polyisocyanurate,
wherein
the base comprises one or more base(s) selected from the group consisting of
a base comprising an alkali metal cation and/or an ammonium cation and having a pK_{b} value at 25°C of from 1 to 10, which preferably does not comprise primary, secondary and or tertiary amino groups,
and
a strong inorganic base having a pK_{b} value at 25°C of < 1.

2. The method of claim 1,
**characterized in that** the hydrolysis is carried out by contacting the polyisocyanurate with water in the presence of a base-catalyst-combination comprising the base and a catalyst,
wherein
the base-catalyst-combination is selected from the group consisting of base-catalyst-combinations (I), (II) or (III),
and wherein
the base-catalyst-combination (I) comprises the base comprising an alkali metal cation and/or an ammonium cation and having a pK_{b} value at 25°C of from 1 to 10, which preferably does not comprise primary, secondary and or tertiary amino groups, and a catalyst selected from the group consisting of quaternary ammonium salts containing an ammonium cation containing 6 to 30 carbon atoms and organic sulfonates containing at least 7 carbon atoms,
and wherein
the base-catalyst-combination (II) comprises the strong inorganic base having a pK_{b} value at 25°C of < 1, and as catalyst a quaternary ammonium salt containing an ammonium cation containing 6 to 14 carbon atoms, preferably 6 to 12 carbon atoms if the ammonium cation comprises a benzyl residue,
and wherein
the base-catalyst-combination (III) comprises the strong inorganic base having a pK_{b} value at 25°C of < 1, and as catalyst a quaternary ammonium salt containing an ammonium cation containing 15 to 30 carbon atoms, preferably 15 to 28, more preferred 15 to 24, even more preferred 16 to 22 and most preferred 16 to 20.

3. The method of claim 1 or 2,
**characterized in that** the base comprising an alkali metal cation comprising and having a pK_{b} value at 25°C of from 1 to 10, is selected from the group consisting of alkali metal phosphates, alkali metal hydrogen phosphates, alkali metal carbonates, alkali metal silicates, alkali metal hydrogen carbonates, alkali metal acetates, alkali metal sulfites, ammonium hydroxide, and mixtures thereof.

4. The method of claim 3 wherein the alkali metals are selected from the group consisting of Na, K and Li and mixtures thereof, most preferred Na and K and mixtures thereof.

5. The method of claim 1 or 2,
**characterized in that** the strong inorganic base is selected from the group consisting of alkali metal hydroxides, alkali metal oxides, alkaline earth metal hydroxides, alkaline earth metal oxides and mixtures thereof.

6. The method of claim 5 wherein the alkali metals are selected from the group consisting of Na, K and Li and mixtures thereof, most preferred Na and K and mixtures thereof and/or the alkaline earth metals are selected from the group consisting of Be, Mg, Ca, Sr, Ba and mixtures thereof, preferably Mg and Ca and mixtures thereof.

7. The method of any one of claim 2 to 6 wherein the phase transfer catalyst is a quaternary ammonium salt having the general structure R₁ R₂ R₃ R₄ NX wherein R₁,R₂,R₃, and R₄ are the same or different and are hydrocarbyl groups selected from alkyl, aryl, and arylalkyl and X is selected from the group consisting of halide, preferably chloride and/or bromide, hydrogen sulfate, alkyl sulfate, preferably methylsulfate and ethylsulfate, carbonate, hydrogen carbonate, carboxylate, preferably acetate, or hydroxide.

8. The method of claim 7, wherein
for base-catalyst-combination (I) and (III) the catalyst is a quaternary ammonium salt having the general structure R₁ R₂ R₃ R₄ NX with
- R₁ and R₂ are the same or different and are alkyl groups with 1 to 12, preferably 1 to 10, more preferred 1 to 7, even more preferred 1 to 6, especially preferred 1 to 5 and most preferred 1 to 4 carbon atoms, wherein the alkyl groups may be linear, branched, cyclic, saturated or unsaturated, most preferred are linear, saturated alkyl groups,
- R₃ is selected from the group consisting of alkyl groups with 1 to 12, preferably 1 to 10, more preferred 1 to 7, even more preferred 1 to 6, especially preferred 1 to 5 and most preferred 1 to 4 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and,
- R₄ is selected from the group consisting of alkyl groups with 3 to 12, preferably 3 to 10, more preferred 3 to 7, most preferred 4 to 6 carbon atoms, aryl groups with 6 to 14, preferably 6 to 12, and most preferred 6 to 10 carbon atoms, and aralkyl groups with 7 to 14, preferably 7 to 12, and most preferred 7 to 10 carbon atoms, wherein the alkyl groups may be linear, branched, cyclic, saturated or unsaturated, most preferred linear and saturated, and
- X is selected from the group consisting of halide, preferably chloride and/or bromide, hydrogen sulfate, alkyl sulfate, preferably methylsulfate and ethylsulfate, carbonate, hydrogen carbonate, acetate or hydroxide.
and/or
for base-catalyst-combination (II) the catalyst is a quaternary ammonium salt having the general structure R1 R₂ R₃ R₄ NX with
- R₁ to R₃ are the same or different and are alkyl groups with 1 to 6, preferably 1 to 5, more preferred 1 to 4, even more preferred 1 to 3, especially preferred 1 or 2 and most preferred 1 carbon atoms, wherein the alkyl groups may be linear, branched, cyclic, saturated or unsaturated, most preferred are linear, saturated alkyl groups,
- R₄ is selected from the group consisting of alkyl groups with 3 to 11, preferably 3 to 10, more preferred 3 to 8, most preferred 4 to 6 carbon atoms, aryl groups with 6 to 11, preferably 6 to 10, and most preferred 6 to 8 carbon atoms, and aralkyl groups with 7 to 11, preferably 7 to 10, and most preferred 7 to 9 carbon atoms, wherein the alkyl groups may be linear, branched, cyclic, saturated or unsaturated, most preferred are linear, saturated alkyl groups, and
- X is selected from the group consisting of halide, preferably chloride and/or bromide, hydrogen sulfate, alkyl sulfate, preferably methylsulfate and ethylsulfate, carbonate, hydrogen carbonate, acetate or hydroxide.

9. The method of claim 7 or 8, wherein
for base-catalyst-combination (I)
R₁ to R₄ are selected such that the sum of carbon atoms in the quaternary ammonium cation is 6 to 14, preferably 7 to 14, more preferred 8 to 13
or
R₁ to R₄ are selected such that the sum of carbon atoms in the quaternary ammonium cation is 15 to 30, preferably 15 to 28, more preferred 15 to 24, even more preferred 16 to 22 and most preferred 16 to 20.

10. The method of claim 7 or 8, wherein
for base-catalyst-combination (II)
R₄ is different from a benzyl residue and R₁ to R₄ are selected such that the sum of carbon atoms in the quaternary ammonium cation is 6 to 14, preferably 7 to 14, more preferred 8 to 13
or
R₄ is a benzyl residue, R₁ to R₃ are selected such that the sum of carbon atoms in the quaternary ammonium cation is 6 to 12, preferably 7 to 12, more preferred 8 to 11.

11. The method of any one of claims 2 to 10 wherein at least 0.5 weight percent of the quaternary ammonium salt is used as phase transfer catalysts, based on the weight of the polyisocyanurate, more preferably 0.5 to 15 weight percent, even more preferred 1 to 10 weight percent, particular preferred more 1 to 8 weight percent, especially preferred 1 to 7 and most preferred 2 to 6 weight percent.

12. The method of any one of claims 1 to 11, wherein the hydrolysis is carried out
at a temperature of from 90°C to 220°C, preferably 100°C to 210°C, more preferred 110°C to 200°C and most preferred 120°C to 190°C
and/or
for 30 minutes to 20 hours, preferably 30 minutes to 16 hours, more preferred 30 minutes to 14 hours, even more preferred 45 minutes to 10 hours, particular preferred 60 minutes to 8 hours, and most preferred 60 minutes to 6 hours
and/or
at atmospheric pressure or under elevated pressure, in particular under a pressure of from 1 to 30 bara, preferably 2 to 20 bara, more preferred 3 to 15 bara.

13. The method of any one of claims 1 to 12,
**characterized in that** the polyisocyanurate is produced by reacting
a) a polyesterpolyol or a mixture of polyesterpolyols or a mixture of polyester- and polyetherpolyol, comprising isocyanate-reactive groups selected from OH groups, SH groups, NH groups, NH₂ groups and mixtures thereof, preferably OH groups, with
b) an excess of one or more isocyanate(s) selected from the group consisting of organic isocyanate, mixture of organic isocyanates, organic polyisocyanate, mixture of organic polyisocyanates and a mixture of organic isocyanate(s) and organic polyisocyanate(s), in the presence of
c) a catalyst catalyzing the reaction of the isocyanate reactive groups with the isocyanate groups and / or the reaction of isocyanate groups with each other, with the proviso that at least one trimerization catalyst is comprised
d) optionally a foam stabilizer
e) optionally a propellant
f) optionally further additives.

14. The method of any one of claims 1 to 13,
**characterized in that** the polyesterpolyol is produced by reacting
one or more aromatic or aliphatic carboxylic acid or a mixture of aromatic and aliphatic carboxylic acids, wherein the carboxylic acids having 2 or 3, more preferred 2 carboxylic acid groups and having 2 to 12, preferably 4 to 10, more preferred 6 to 10 carbon atoms per molecule,
with
a polyol or a mixture of polyol(s), wherein at least one polyol having 2 or more isocyanate-reactive groups selected from group consisting of OH groups, SH groups, NH groups, NH₂ groups and mixtures thereof, preferably OH groups, and wherein the polyol or the mixture of polyol(s) in sum having on an average 1.8 to 8, preferably 1.9 to 5, more preferred 2 to 3 and most preferred 2 of said isocyanate-reactive groups, and wherein the polyol(s) having on an average 2 to 12, preferably 2 to 10, more preferred 2 to 6 carbon atoms and wherein the polyol(s) may comprise ether and/or carbonate functional groups, preferably polyetherpolyol or polyethercarbonatpolyol.

15. The method according to claim 14,
**characterized in that**
the aromatic carboxylic acid(s) is/are selected from the group consisting of phthalic acid, preferably (ortho)-phthalic acid, isophthalic acid, terephthalic acid and isomeric naphthalene dicarboxylic acids
and/or
the aliphatic carboxylic acid(s) is/are selected from the group consisting of succinic acid, glutaric acid, adipic acid, suberic acid, azelaic acid, sebacic acid, decandicarboxylic acid, maleic acid and fumaric acid.

16. The method according to claim 1 to 15,
**characterized in that**
at least 90%, preferably at least 95%, more preferably at least 98% of the polyol components used to prepare the polyisocyanurate are polyesterpolyols having a hydroxyl number of 100 to 450 mg KOH / g, preferably 120 to 400 mg KOH / g, more preferably 140 to 350 mg KOH / g,
and/or
that the organic isocyanate and/or polyisocyanate(s) is/are selected from the group consisting of monomeric, oligomeric or polymeric, aliphatic, cycloaliphatic, aryl aliphatic or aromatic, isocyanates with 2 or more, preferably 2 to 4 isocyanate groups and mixtures thereof,
and/or
that the polyisocyanurate has an isocyanate index of equal to or more than 150, preferably >180, more preferred > 250, most preferred > 250 to 500,
and/or
that the polyisocyanurate is foamed, preferably a rigid foam.

17. The method of any one of claims 1 to 16,
**characterized in that**
the one or more carboxylic acid(s) obtained after the hydrolysis is selected from the group consisting of phthalic acid, preferably (ortho)-phthalic acid, terephthalic acid, isophthalic acid, and isomeric naphthalene dicarboxylic acids, succinic acid, glutaric acid, adipic acid, suberic acid, azelaic acid, sebacic acid, decandicarboxylic acid, maleic acid, fumaric acid and mixtures thereof
and/or
the one or more polyol(s) obtained after the hydrolysis is a diol on the basis of glycol and/or glycol ether having a molecular weight below 180 g/mol, preferably below 140 g/mol, particular preferred monoethylene glycol and/or diethylene glycol,
and/or
the one or more organic amine and/or polyamine obtained after the hydrolysis is selected from the group consisting of dodecane 1,12-diamin, 2-ethyltetramethylene 1,4-diamine, 2-methylpentamethylene 1,5-diamine, tetramethylene 1,4-diamine, pentamethylene diamine (PDA) and preferably hexamethylene 1,6-diamine (HMDA), cycloaliphatic diamines such as cyclohexane 1,3- and 1,4-diamine and also any mixtures of these isomers, 4,4'-Methylenedicyclohexyldiisocyanate (H12MDI), 1-isocyanato-3,3,5-trimethyl-5-isocyanatomethylcyclohexane (isophorone diamine or IPDA for short), hexahydrotolylene 2,4- and 2,6-diamine and also the corresponding isomer mixtures, and preferably aromatic diamines and polyisocyanates, for example toluene 2,4- and 2,6-diamine (TDA) and the corresponding isomer mixtures, naphthalene diamine, diethyltoluene diamine, mixtures of diphenylmethane 4, 4', 2,4'- and 2,2'-diamines (MDA) and polyphenylpolymethylene polyamines and their higher condensed analogues with an average functionality of 2 to 4.

18. The method of any one of claims 1 to 17 comprising an additional step of separating and recovering and optionally purifying the reaction products of the hydrolysis, preferably the separation and purification methods are selected from the group consisting of filtration, membrane separation, phase separation, chromatographic methods, distillation, extraction and combinations of said methods.

19. Use of the one or more carboxylic acid(s) and/or polyol(s) and/or organic amine(s) and/or polyamine(s) obtained with the process according to any one of claims 1 to 18 for production of new chemical, preferably polyurethane, preferably polyurethane foam, or polyisocyanurates, preferably polyisocyanurate foams.
